# EUROPÄISCHE PATENTSCHRIFT

(11) **EP 2 246 342 B1**
(45) Veröffentlichungstag und Bekanntmachung des Hinweises auf die Patenterteilung: **23.01.2013**
(21) Anmeldenummer: 10160157.3
(22) Anmeldetag: 16.04.2010
(51) Int. Cl.: C07D 323/00, C07D 323/04, C07K 16/44, G01N 33/53

(54) **Triacetontriperoxid-Derivat, Verfahren zu dessen Herstellung sowie dessen Verwendung**
Triacetone triperoxide derivative, method for its manufacture and its application
Dérivé de triperoxyde de triacétone, procédé destiné à sa fabrication et son utilisation

(30) Priorität: 27.04.2009 DE 102009002652
(43) Veröffentlichungstag der Anmeldung: 03.11.2010
(73) Patentinhaber: BAM Bundesanstalt für Materialforschung und -prüfung, 12205 Berlin (DE)
(72) Erfinder: Walter, Astrid, 12524, Berlin (DE); Schneider, Dr. Rudolf, 14169, Berlin (DE); Weller, Dr. Michael G., 15834 Rangsdorf (DE)
(74) Vertreter: Zimmermann & Partner

(56) Entgegenhaltungen:
- US-A- 5 367 061
- US-B1- 6 767 717
- MASAHIRO MIURA ET AL: "Synthesis of 1,4-disubstituted (or 1,4,4-trisubstituted) 2,3,5,6,11-pentaoxabicyclo[5.3.1]undecanes ", JOURNAL OF THE CHEMICAL SOCIETY, CHEMICAL COMMUNICATIONS, CHEMICAL SOCIETY. LETCHWORTH, GB, Nr. 24, 1. Januar 1980 (1980-01-01), Seiten 1279-1281, XP008141336, ISSN: 0022-4936, DOI: 10.1039/C39800001279
- R SCHULTE-LADBECK: "Determination of triacetonetriperoxide in ambient air", ANALYTICA CHIMICA ACTA, Bd. 482, Nr. 2, 15. April 2003 (2003-04-15), Seiten 183-188, XP55005204, ISSN: 0003-2670, DOI: 10.1016/S0003-2670(03)00212-5
- SMITH RICHARD G ET AL: "A review of biosensors and biologically-inspired systems for explosives detection", ANALYST, ROYAL SOCIETY OF CHEMISTRY, GB, Bd. 133, Nr. 5, 1. Mai 2008 (2008-05-01), Seiten 571-584, XP008094172, ISSN: 0003-2654, DOI: 10.1039/B717933M
- BOWEN J ET AL: "GAS-PHASE DETECTION OF TRINITROTOLUENE UTILIZING A SOLID-PHASE ANTIBODY IMMOBILIZED ON A GOLD FILM BY MEANS OF SURFACE PLASMON RESONANCE SPECTROSCOPY", APPLIED SPECTROSCOPY, THE SOCIETY FOR APPLIED SPECTROSCOPY. BALTIMORE, US, Bd. 57, Nr. 8, 1. August 2003 (2003-08-01) , Seiten 906-914, XP009055409, ISSN: 0003-7028, DOI: 10.1366/000370203322258850

## Beschreibung

Die Erfindung betrifft ein Triacetontriperoxid- Derivat, ein Verfahren zu dessen Herstellung sowie dessen Verwendungen zur Herstellung von Antikörpern und zur Herstellung von lmmunsensoren zum Nachweis von TATP, insbesondere in der Luft.

Triacetontriperoxid (TATP) - oder korrekt nach IUPAC 3,3,6,6,9,9-Hexamethyl-1,2,4,5,7,8-hexaoxocyclononan (CAS-Nr. 17088-37-8) - wurde erstmalig bereits 1895 aus Aceton CH₃COCH₃ und Wasserstoffperoxid H₂O₂ in Gegenwart geringer Mengen Phosphorsäure synthetisiert (R. Wolffenstein "Über die Einwirkung von Wasserstoffsuperoxyd auf Aceton und Mesityloxyd", Berichte der Deutschen Chemischen Gesellschaft, 28(2), 1895, 2265-2269). Bereits damals wurde die enorme, durch Schlag, Reibung oder Erwärmung auslösbare Explosivität der Verbindung beobachtet.

Heute ist TATP weitläufig als Explosivstoff bekannt, der u.a. von Selbstmordattentätern eingesetzt wird. Seine Bevorzugung für terroristische Anwendungen liegt einerseits darin, dass die Verbindung sehr einfach aus allgemein verfügbaren Ausgangsstoffen hergestellt werden kann, etwa aus in Nagellackentfernern enthaltenem Aceton und Wasserstoffperoxid, das sich in Haarbleichmitteln findet. Durch seine Eigenschaft als Initialsprengstoff, d.h. durch seine hohe Stoß- und Schlagempfindlichkeit, entfällt zudem das Erfordernis eines gesonderten, gegebenenfalls metallhaltigen und damit leicht detektierbaren Zünders. Darüber hinaus enthält TATP keinen Stickstoff, wodurch einige Detektionsverfahren für seinen Nachweis ausfallen, und hat eine ähnlich niedrige Dichte wie Zucker und wird dadurch von gängigen, z.B. bei der Gepäckdurchleuchtung eingesetzten Scannern nicht angezeigt.

Neben seinem kriminellen Einsatz wurde TATP jedoch auch wiederholt beabsichtigt oder unbeabsichtigt von Hobbybastlern hergestellt oder entsteht gelegentlich durch unsachgemäße gemeinsame Entsorgung entsprechender Vorstufen in Chemikalienabfällen.

Es besteht somit ein dringender Bedarf an einem Verfahren bzw. einer Detektionsvorrichtung zum Nachweis von TATP in der Luft, auf Oberflächen von Gegenständen, in Geweben etc..

Für eine hochselektive und sehr nachweisempfindliche Detektion von Substanzen, insbesondere auch Explosivstoffen, sind immunchemische Methoden besonders geeignet. Sie beruhen auf einer starken Bindung des hier als Antigen agierenden Analyten an einen hochselektiven Antikörper und einem geeigneten Nachweis dieser Bindung durch Umwandlung des Bindungsvorgangs in ein einfach nachzuweisendes Signal. Beispielsweise ist bekannt, die Antikörper auf einer Quarzkristall-Mikrowaage zu verwenden (z.B. A.V. Kuznetsov und O.I. Osetrov in "Detection and Disposal of Improvised Explosives" NATO Security through Science Series - B: Physics and Biophysics, H. Schubert und A. Kuznetsov (Hrsg.), Springer-Verlag, 2006, 7-23). Ein Gassensor mit einer Nachweisempfindlichkeit für Trinitrotoluol (TNT) von 73 ppb, der mit monoklonalen TNT-Antikörpern ausgestattet ist und die Methode der Oberflächenplasmonenresonanz (SPR) zum Nachweis der Antikörper-Antigen-Bindung nutzt, wurde in der Literatur beschrieben (J. Bowen et al. "Gas-Phase Detection of Trinitrotoluene Utilizing a Solid-Phase Antibody Immobilized on a Gold Film by Means of Surface Plasmon Resonance Spectroscopy", Applied Spectroscopy 57(8), 2003, 906-914).

Der Dampfdruck von TATP bei Raumtemperatur wurde gaschromatographisch zu etwa 7 Pa bestimmt und ist damit um einen Faktor 10⁴ höher als der von TNT (J.C. Oxley et al. "Determination of the Vapor Density of Triacetone Triperoxide (TATP) Using a Gas Chromatagraphy Headspace Technique", Propellants Explosives Pyrotechnics 30(2), 2005, 127-130). Somit sollte eine Detektion von TATP in der Luft grundsätzlich möglich sein. Jedoch ist ein Immunsensor für TATP bislang ebenso wenig bekannt wie ein TATP-Antikörper (A.V. Kuznetsov und O.I. Osetrov in "Detection and Disposal of Improvised Explosives" NATO Security through Science Series - B: Physics and Biophysics, H. Schubert und A. Kuznetsov (Hrsg.), Springer-Verlag, 2006, 7-23).

Darüber hinaus ist auch Diacetondiperoxid (DADP, CAS-Nr. 1073-91-2, IUPAC-Name 3,3,6,6,-Tetramethyl-1,2,4,5-tetraoxan) bekannt, das über ähnliche Eigenschaften wie sein trimeres Pendant verfügt und sich in Gegenwart bestimmter Säuren in einer äußerst langsamen Reaktion aus diesem bildet (F. Dubnikova et al. "Decomposition of triacteone triperoxide is an entropic explosion", Journal of the American Chemical Society 2005, 127(4), 1146-1159; R. Matyas "Study of TATP: Spontaneous transformation of TATP to DADP" Propellants Explosives Pyrotechnics 33(2), 2008, 89-91).

Antikörper werden durch Immunisierung von Wirbeltieren gewonnen, wozu diesen üblicherweise das Antigen injiziert wird. Allerdings muss im Fall von niedermolekularen Analyten wie TATP oder DADP, damit dieses vom Immunsystem des Tieres als Antigen erkannt wird, die Substanz an ein (immunogenes) Trägerprotein gekoppelt werden. Aufgrund seiner chemischen Struktur, insbesondere des Fehlens geeigneter chemischer Gruppen, ist eine Kopplung von TATP bzw. DADP an Proteine jedoch nicht ohne Weiteres möglich.

Der Erfindung liegt die Aufgabe zugrunde, ein Derivat von TATP zur Verfügung zu stellen, das an ein Protein koppelbar ist, um somit TATP- spezifische Antikörper zum Einsatz in TATP- Sensoren herstellen zu können. Folglich besteht eine weitere Aufgabe der Erfindung in der Bereitstellung in TATP- spezifischen Antikörpern und Sensoren.

Diese Aufgabe wird durch ein Triacetontriperoxid-Derivat gemäß der allgemeinen Formel (I) gelöst, 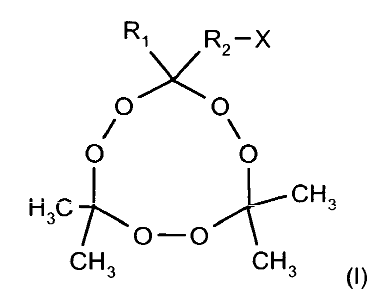 worin R₁ ein Wasserstoffrest H oder eine optional halogenierte oder perhalogenierte C1- bis C5-Alkylgruppe ist, R₂ ein Linker-Molekül bedeutet und X eine reaktive oder aktivierbare Gruppe, die kovalent oder nicht-kovalent (d.h. über physikalische Wechselwirkungen) an Proteine koppelbar ist (z.B. Greg. T. Hermanson: "Bioconjugate Techniques" 2. überarbeitete Auflage (2008), Academic Press).

Die erfindungsgemäßen Derivate (die beide Enantiomere oder das Racemat umfassen können) unterscheiden sich von der eigentlichen Target-Struktur des TATP durch die Substitution eines Methylrestes durch eine, die funktionelle Gruppe X enthaltende Seitenkette. Durch diese funktionelle Gruppe ist eine Kopplung des Derivates an ein (immunogenes) Trägerprotein möglich, wobei die insbesondere kovalente Bindung beispielsweise an eine funktionelle Gruppe einer Seitenkette des Proteinrückgrates, etwa einer Carboxy-, Hydroxyl-, Amino- oder Thiolgruppe erfolgen kann.

Überraschend an den erfindungsgemäßen Derivaten ist unter anderem ihre für diese Peroxide ungewöhnliche Stabilität, die auch eine Immunisierung ermöglicht. Aufgrund dieser längerfristigen Stabilität ist auch eine kommerzielle Nutzung z.B. von Konjugaten in einfacher Weise möglich. Die Substanzen stellen praktisch keine Gefahr für Mensch und Umwelt dar und lassen sich verdünnt gefahrlos transportieren.

Bei dem Linker-Molekül R₂ kann es sich um nahezu beliebige Strukturen handeln, die beispielsweise aus der Fachliteratur der Immunologie bekannt sind (z.B. Greg. T. Hermanson: Bioconjugate Techniques). Insbesondere kann R₂ ein vorzugsweise unverzweigter, gesättigter oder einfach oder mehrfach ungesättigter C1- bis C20-Alkylrest sein. Dabei werden unter "einfach oder mehrfach ungesättigten Alkylgruppen" solche Kohlenwasserstoffreste verstanden, die eine oder mehrere Doppelbindungen oder Dreifachbindungen oder Mischungen von diesen in beliebigen Anordnungen aufweisen. Außerdem kann R₂ ein Oligoethylenoxid, ein Peptid, ein Peptidoid, eine Nukleinsäure, ein Oligozucker sein sowie andere synthetische oder natürliche Polymere oder Copolymere und Kombinationen der vorgenannten Gruppen.

Gemäß einer bevorzugten Ausgestaltung kann R₂ vorteilhaft ein unverzweigter, gesättigter C1- bis C8-Alkylrest sein, insbesondere ein C2- bis C7-Alkylrest, vorzugsweise ein C5-Alkylrest.

Für die koppelbare (aktive) Gruppe X kommen gleichfalls aus der einschlägigen Fachliteratur bekannte funktionelle Gruppen in Frage (z.B. Greg. T. Hermanson: Bioconjugate Techniques). Insbesondere kann X eine Aminogruppe NH₂, eine Carboxylgruppe COOH, eine Aldehydgruppe CHO, eine Hydroxylgruppe OH, eine Thiolgruppe SH, eine Oxirangruppe (Epoxidgruppe) CH(O)CH₂, eine Aziridingruppe (Ethylenimingruppe) CH(NH)CH₂, eine Hydrazingruppe NH-NH₂, eine Hydrazidgruppe CONH-NH₂, eine Hydroxylamonogruppe ONH₂, ein N-Hydroxysuccinimidester u.ä. bedeuten. Ebenfalls eingeschlossen sind Salze, Ester (insbesondere aktivierte Ester) und Anhydride der funktionellen Gruppen X, soweit sie zu deren Bildung befähigt sind. Auch nicht-kovalente Bindungsgruppen sind als koppelbare Gruppe X geeignet, so z.B. Biotin und dessen Derivate, so genannte Peptid-Tags, insbesondere His-Tags (Oligo-Histidin) und andere. Die Gruppe X kann auch eine detektierbare Markierung sein oder eine solche umfassen, beispielsweise ein Farbstoff, insbesondere Fluoreszenzfarbstoff oder ein anderer, eine radioaktive Markierung oder eine stabile Isotopen-Markierung.

Es ist ferner bevorzugt, dass X eine Carboxylgruppe oder ein aktivierter Ester, ein gemischtes oder symmetrisches Anhydrid ist. Die Verknüpfung einer solchen Carboxylgruppe an ein Trägerprotein ist besonders einfach durchführbar.

In bevorzugter Ausgestaltung der Erfindung ist R₁ eine Methylgruppe.

Vorzugsweise entspricht das erfindungsgemäße Triacetontriperoxid-Derivat der speziellen Formel (la) (6-(3,6,6,9,9-Pentamethyl-1,2,4,5,7,8-hexaoxonan-3-yl)hexansäure) oder einem aktivierten Ester (z.B. N-Hydroxysuccinimidester, NHS-Ester) oder einem gemischten Anhydrid (z.B. hergestellt mit Isobutylchlorformiat).

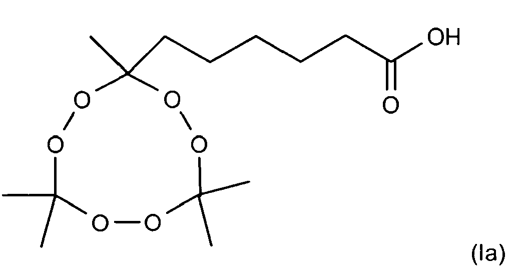

Ein weiterer Aspekt der Erfindung betrifft ein Verfahren zur Herstellung des erfindungsgemäßen, vorstehend beschriebenen Triacetontriperoxid- Derivats, wobei Aceton, Wasserstoffperoxid und eine Verbindung gemäß der allgemeinen Formel (III) miteinander umgesetzt werden, 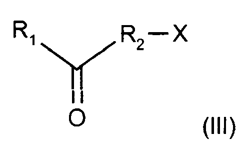 worin R₁ ein Wasserstoffrest H oder eine optional halogenierte oder perhalogenierte C1- bis C5-Alkylgruppe ist, R₂ ein Linker-Molekül bedeutet und X eine funktionelle, kovalent oder nicht-kovalent koppelbare Gruppe.

Bevorzugte Ausgestaltungen der Reste R₁ und R₂ entsprechen den im Zusammenhang mit dem erfindungsgemäßen TATP-Derivat genannten.

Die Herstellung des TATP-Derivats entspricht somit der Reaktionsgleichung (IV).

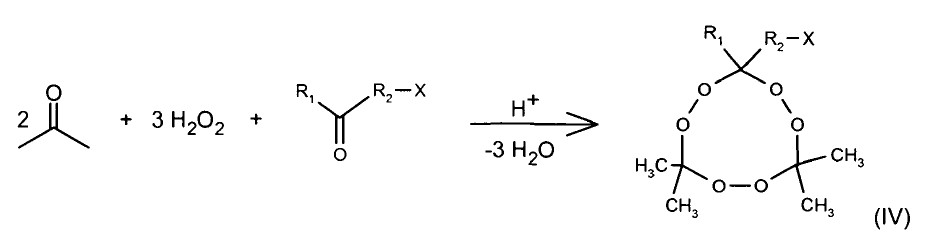

Vorzugsweise wird als Verbindung gemäß allgemeiner Formel (III) 7-Oxooktansäure gemäß Formel (IIIa) eingesetzt, so dass das Triacetontriperoxid-Derivat nach Formel (la) resultiert.

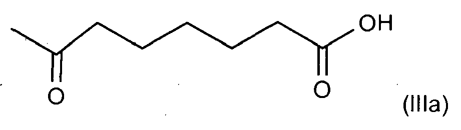

Die hier angewandte Reaktion entspricht somit dem Ansatz gemäß Wolffenstein (s.o.), wobei ein Aceton-Baustein durch die Verbindung gemäß allgemeiner Formel (III) und insbesondere durch 7-Oxooktansäure ersetzt wird.

Die erfindungsgemäße Reaktion wird durch die Gegenwart von Protonen wesentlich beschleunigt. Daher wird sie vorzugsweise in einem sauren Milieu durchgeführt. Dieses kann durch Zugabe laborüblicher Mineralsäuren erreicht werden, beispielsweise Schwefelsäure, Salzsäure, Salpetersäure, Phosphorsäure, Perchlorsäure, Methansulfonsäure, Ameisensäure, Essigsäure, Zitronensäure, oder andere. Vorzugsweise wird hier Schwefelsäure oder Salzsäure eingesetzt. Falls eines der Edukte selbst saure Eigenschaften aufweist, so kann auf die Zugabe einer Säure verzichtet und die Reaktion autokatalytisch durchgeführt werden.

Besonders gute Ausbeuten und geringere Mengen Nebenprodukte werden erhalten, wenn Aceton, Wasserstoffperoxid und die Verbindung gemäß der allgemeinen Formel (III) in einem molaren Verhältnis von etwa 1 : 1 : 1 zum Ansatz gebracht werden. Auf diese Weise können die Anteile an Nebenprodukten geringer gehalten werden.

Abgesehen von aus dem Wasserstoffperoxid stammendem Wasser kann die Reaktion ohne die Zugabe von Lösungsmitteln durchgeführt werden, da Aceton selbst ein Lösungsmittel ist.

Die Reaktion wird vorzugsweise bei Zimmertemperatur oder niedriger durchgeführt.

Ein weiterer Aspekt der vorliegenden Erfindung betrifft die Verwendung des vorstehend beschriebenen erfindungsgemäßen TATP- Derivats zur kovalenten oder nicht-kovalenten Kopplung an Proteine (z.B. immunogene Carrier-Proteine, Beschichtungsproteine oder Markerenzyme), DNA, Zucker, oder Labels (z.B. Fluoreszenzfarbstoffe, Radioisotope oder stabile Isotope), inerte Träger (z.B. Agarose), Mikropartikel oder Membranen.

Noch ein weiterer Aspekt der vorliegenden Erfindung betrifft die Verwendung des vorstehend beschriebenen erfindungsgemäßen TATP- Derivats als Antigen zur Herstellung von TATP- spezifischen Bindern, insbesondere von Antikörpern, vorzugsweise von monoklonalen Antikörpern, deren Fragmenten, Derivaten oder Fusionsprodukten, Antikörper-Analogen, Aptameren, Bindern auf Basis von Proteingerüsten, molekular geprägte Systeme (MIPs), nanostrukturierte Materialien, selektiv bindenden Oberflächen oder isotopenmarkierten Verbindungen. Die Herstellung von Antikörpern erfolgt üblicherweise durch vorzugsweise kovalente Bindung des Derivats an ein geeignetes Trägerprotein, beispielsweise Ovalbumin, Rinderserumalbumin oder KLH (Hämocyanin), und anschließende Immunisierung von Wirbeltieren. Hierzu werden diese dem Antigen, d.h. dem Konjugat aus Trägerprotein und daran gekoppelten TATP- Derivat, ausgesetzt, was üblicherweise durch mehrfache Injektion des Antigens erfolgt. Nach einer gewissen Immunisierungsdauer können dann die gebildeten (polyklonalen) Antikörper aus dem Blut des Tieres isoliert und aufgereinigt werden. Monoklonale Antikörper können ebenfalls aus derartig immunisierten Tieren gewonnen werden. Die zur Herstellung von polyklonalen und monoklonalen Antikörpern angewandten Methoden gehören zum allgemeinen Fachwissen von Biochemikern und Molekularbiologen und müssen daher hier nicht im Einzelnen erläutert werden.

Die so erhaltenen TATPspezifischen Binder, insbesondere die Antikörper, werden bevorzugt in einem Affinitätssensor (Immunsensor) zum Nachweis von Triacetontriperoxid (TATP) eingesetzt. Neben den immobilisierten Antikörpern enthält ein solcher Sensor einen Detektionsmechanismus, der bei erfolgter Bindung zwischen Antikörper und TATP (oder einem Derivat von diesen) ein messbares Signal liefert. Hier kommen grundsätzlich alle bei bekannten Immunsensoren angewandten Detektionssysteme in Frage, insbesondere Quarzmikrowaagen, Cantilever-Systeme, SPR-Systeme (surface plasmon resonance) und andere optische und elektrochemische Systeme.

Ebenfalls können die TATP- spezifischen Binder, insbesondere die Antikörper, in Affinitätsassays (z.B. ELISA) oder in affinitätsanreichernden Systemen (z.B. Immunaffinitätschromatographiesäulen) zum Nachweis von Triacetontriperoxid (TATP) eingesetzt werden.

Die für das erfindungsgemäße Triacetontriperoxid- Derivat und damit für TATP P selbst spezifischen Antikörper, insbesondere monoklonalen Antikörper, oder Antikörperfragmente sowie ein solche Antikörper enthaltender Sensor zum Nachweis von TATP stellen weitere Aspekte der vorliegenden Erfindung dar.

Weitere Anwendungen der Antikörper betreffen Immunoassays (z.B. ELISA) für die Detektion von TATP in flüssigen Proben; Affinitätsmaterialien, die immobilisierte Antikörper oder Haptene enthalten, für die Affinitätschromatographie oder Affinitätsanreicherung, und immunologische Schnelltests zum Nachweis von TATP , beispielsweise in Form von Teststreifen, immunchromatographischen Systemen, Lateral-Flow-Tests oder ähnliche Systeme.

Darüber hinaus können andere spezifisch mit TATP wechselwirkende "Binder" hergestellt werden. Solche Verbindungen sind beispielsweise Antikörperfragmente (z.B. Fab), rekombinante Antikörper, DNA- oder RNA-Aptamere, Binder auf Basis anderer Polymergerüste ("protein scaffolds"), Molecularly Imprinted Polymers (MIPs), Phage Display Peptide, Mimotope.

Ebenfalls können mit Hilfe der erfindungsgemäßen Derivate weitere TATP- Tracer synthetisiert werden, etwa Enzymkonjugate, Fluoreszenzkonjugate, Radiotracer, Isotopentracer, Partikeltracer u.a.

Eine weitere Verwendung besteht in der Herstellung von Microarrays (Immunoarrays oder Hapten-Arrays) oder Bead-based Arrays sowie die Verwendung der Derivate zur Zellmarkierung in der Durchflusszytometrie.

Weitere bevorzugte Ausgestaltungen der Erfindung ergeben sich aus den übrigen, in den Unteransprüchen genannten Merkmalen.

Die Erfindung wird nachfolgend in Ausführungsbeispielen anhand der zugehörigen Figuren näher erläutert. Es zeigen:
- Figur 1: MALDI-TOF-Massenspektren eines an BSA gekoppelten TATP-Derivats (durch- gezogene Linie) mit einer Kopplungsdichte von 11 TATP-Derivat-Molekülen pro BSA-Molekül sowie von ungekoppeltem BSA (gestrichelte Linie);
- Figur 2: MALDI-TOF-Massenspektren eines an BSA gekoppelten TATP-Derivats (durch- gezogene Linie) mit einer Kopplungsdichte von 15 TATP-Derivat-Molekülen pro BSA-Molekül sowie von ungekoppeltem BSA (gestrichelte Linie);
- Figur 3: zeitlicher Verlauf der Immunisierung von Mäusen mit BSA-Konjugat des TATP- Derivats (a: direkter ELISA; b: indirekter ELISA); und
- Figur 4: zeitlicher Verlauf der Immunisierung von Kaninchen mit BSA-Konjugat des TATP-Derivats (a: direkter ELISA; b: indirekter ELISA).

### Beispiel 1:

### 1.1 TATP-Derivat-Synthese

Die Synthese erfolgte gemäß der unten angegebenen Reaktionsgleichung. Es wurden 80,71 mg 7-Oxooktansäure (Reinheit 98%, 0,5 mmol) eingewogen und in 36,76 µl Aceton (0,5 mmol) gelöst und 51,05 µl Wasserstoffperoxid (30 %, 0,5 mmol) zugemischt. Durch Zugabe von 2,5 µl Schwefelsäure H₂S0₄ (2 M, 0,005 mmol) bei 0 °C wurde ein saures Milieu eingestellt (pH-Wert nicht bestimmt, Verhältnis Aceton/Säure ≤ 1x 10⁻²). Der Ansatz wurde für 24 h bis ca. 7 Tage bei Raumtemperatur ruhen gelassen. Danach wurde mit H₂O ausgefällt und ein zähflüssiges Pellet erhalten, das in einer Acetonitril/Wasser-Mischung aufgenommen wurde. Die Aufreinigung erfolgte mittels HPLC-DAD per Acetonitril/Wasser-Gradienten mit TFA.

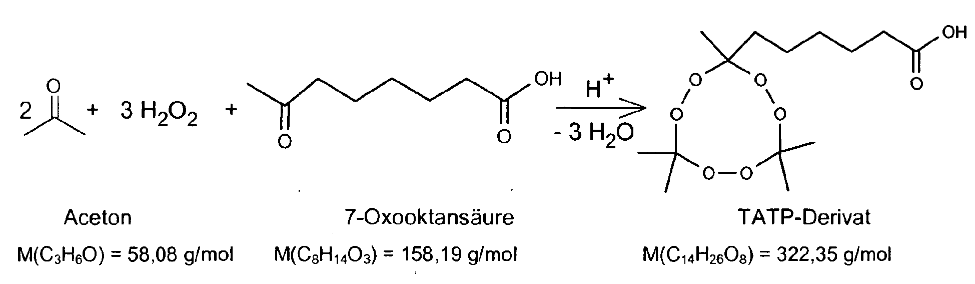

Die Struktur des gereinigten TATP-Derivats wurde ¹H-NMR-spektroskopisch (Bruker, 600 MHz) und ¹³C-NMR-spektroskopisch (Bruker 150,9 MHz) jeweils in MeOD verifiziert. Außerdem wurde ein hochaufgelöstes Massenspektrum (ESI-negative-FT-MS) des gereinigten TATP-Derivats in Acetonitril aufgenommen. Die gefundenen Massen von 321,1552 (m/z) weicht um weniger als 0,0001 % von der theoretischen (M(C₁₄H₂₅O₈) = 321,1555) ab.

### 1.2 Synthese des aktivierten Esters

Nach dem folgenden Reaktionsschema wurde das TATP-Derivat mit N-Hydroxysuccinimid (NHS) und Dicyclohexylcarbodümid (DCC) in wasserfreiem THF oder wasserfreiem DMF in einem molaren Verhältnis TATP-Derivat : NHS : DCC von 1 : 1,2-2 : 1,2-2 umgesetzt. Es ist hierbei strengstens auf wasserfreie Bedingungen zu achten. Dabei wurde der aktivierte Ester des TATP-Derivats erhalten.

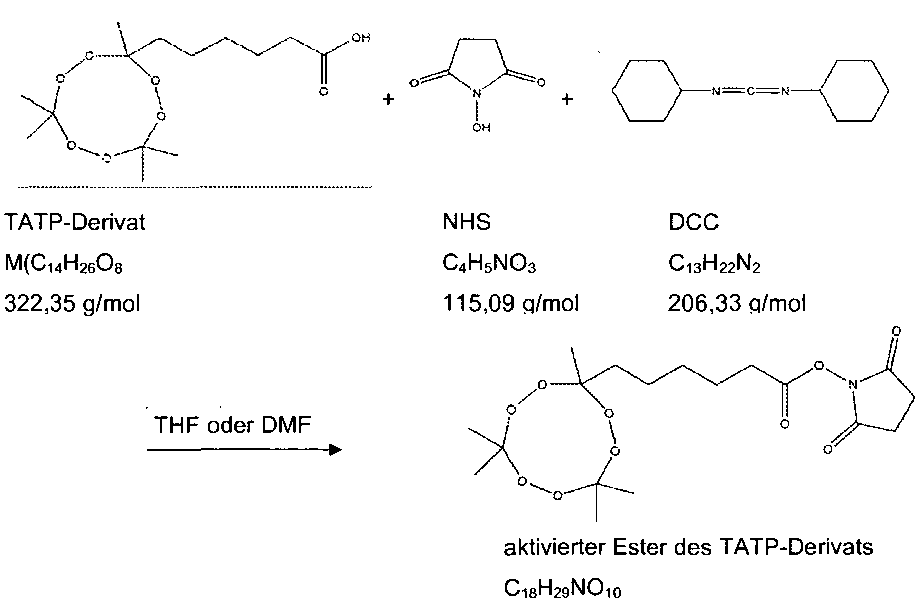

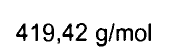

### 1.3 Kopplung des aktivierten Esters an ein Protein

Der gemäß der vorstehenden Beschreibung hergestellte aktivierte Ester des TATP-Derivats wurde in Gegenwart von 0,13 M Natriumhydrogencarbonat NaHC0₃ (0,13 M) an verschiedene Proteine gekoppelt. Es wurden die Proteine Rinderserumalbumin (BSA, ca. 66 kDa) Meerrettichperoxidase (POD, ca. 44 kDa) und Ovalbumin (OVA, ca. 43 kDa) eingesetzt. Es entstand jeweils eine kovalente Peptidbindung über ein Lysin des jeweiligen Proteins. Das nachfolgende Reaktionsschema zeigt die Kopplungsreaktion am Beispiel des BSA.

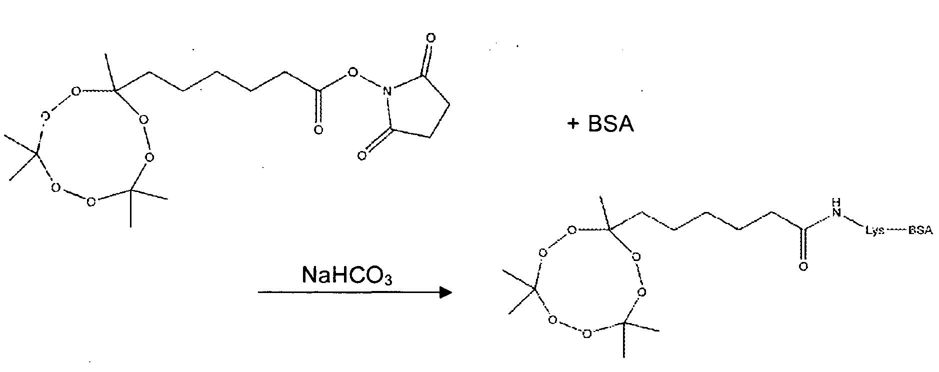

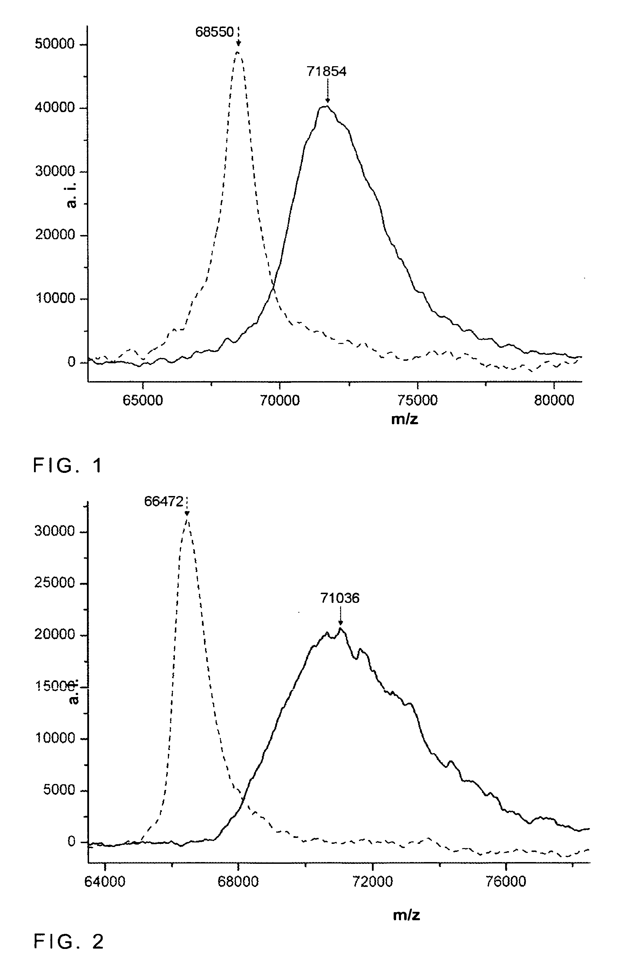

Als Nachweis des Erfolgs der Kopplung des TATP-Derivats an das Protein wurden MALDI-TOF-MS-Spektren zweier BSA-Konjugate im Vergleich mit ungekoppeltem BSA aufgenommen. Das Entsalzen der Protein-Lösung vor der Messung erfolgte über ZebaSpin-Säulen. Als Matrix wurde Sinapinsäure verwendet. Die Spektren wurden durch einen gleitenden Durchschnitt (100 Punkte) geglättet und einer Basislinienkorrektur unterzogen. Es wurde keine Massenkalibration des MALDI-TOF-Massenspektrometers durchgeführt. Die Massenspektren sind in Figur 1 gezeigt, in der die gestrichelte Linie das Spektrum von ungekoppeltem BSA (Edukt) zeigt und die durchgezogene Linie das des BSA-Konjugats (= Immunogen) für die Immunisierung von Mäusen, welches eine mittlere Kopplungsdichte von 11 Haptenen (TATP-Derivat-Moleküle) pro BSA-Molekül aufweist. In Figur 2 sind analoge Massenspektren dargestellt, wobei die mittlere Kopplungsdichte hier 15 Haptenen (TATP-Derivat-Moleküle) pro BSA-Molekül beträgt.

Für Kopplungen mit Meerrettichperoxidase (POD) und Ovalbumin (OVA) wurden mittlere Kopplungsdichten von 2 bzw. 4 Haptenen (TATP-Derivat-Moleküle) je Protein-Molekül erzielt (nicht dargestellt).

### 1.4. TATP-Antikörper-Herstellung in Maus

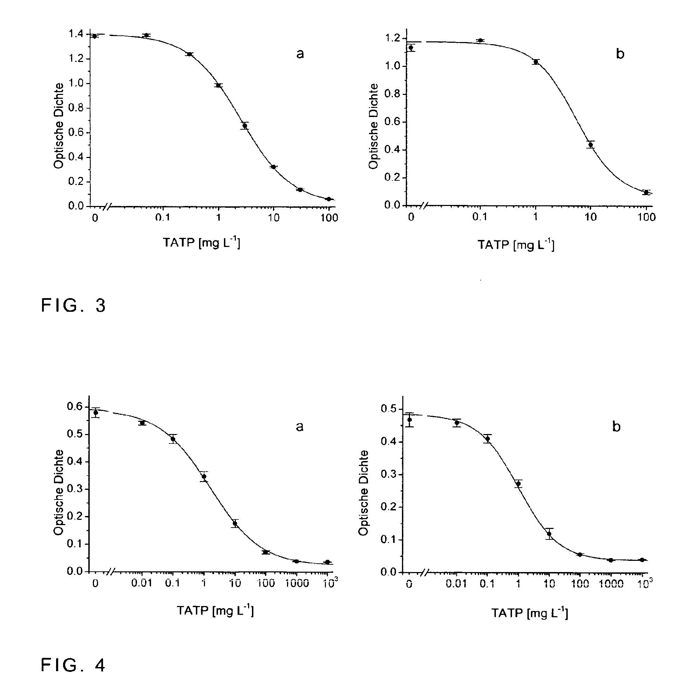

Mit dem unter 1.3 gezeigten BSA-Konjugat wurden 3 BALB/c-Mäuse immunisiert, dazu wurden 50 µg Immunogen alle 4 Wochen injiziert. Die Erstimmunisierung erfolgte unter Verwendung von komplettem Freunds Adjuvans, die Nachimmunisierungen wurden mit inkomplettem Freunds Adjuvans durchgeführt. Mit den Seren, die 7 Tage nach jeder Immunisierung den Tieren entnommen wurden, konnte der Immunisierungsverlauf per ELISA (Enzyme-Linked Immunosorbent Assay) überwacht werden, woraus sich die in Figur 3 gezeigten sigmoidalen TATP-Kalibrierkurven ergaben (Fehlerbalken: Standardabweichung). Kurve a zeigt einen direkten ELISA mit 1:10.000 verdünntem Mausserum und 1:10.000 verdünnten TATP-Peroxidase-Tracer (TATP-Derivat-POD-Konjugat). Der Wendepunkt ist bei 2,5 mg L⁻¹; die mittlere Antikörperaffinität beträgt ca. 5,7 µM und die Nachweisgrenze liegt bei 65 µg L⁻¹ (n = 4). Kurve b zeigt einen indirekten ELISA mit 1:10.000 verdünntem Mausserum und 1:5.000 verdünnten anti-Maus-Peroxidase-Tracer. Der Wendepunkt ist bei 5,6 mg L⁻¹; die mittlere Antikörperaffinität beträgt ca. 13 µM und die Nachweisgrenze liegt bei 870 µg L⁻¹ (n = 3).

### 1.4 TATP-Antikörper-Herstellung in Kaninchen

Mit dem unter 1.3 gezeigten BSA-Konjugat wurden 2 Kaninchen immunisiert, dazu wurden 50 µg Immunogen alle 4 Wochen injiziert. Die Immunisierungen erfolgten unter Verwendung eines Adjuvans. Mit den Seren, die 7 Tage nach jeder Immunisierung den Tieren entnommen wurden, konnte der Immunisierungsverlauf per ELISA überwacht werden, woraus sich die in Figur 4 gezeigten sigmoidalen TATP-Kalibrierkurven ergaben (Fehlerbalken: Standardabweichung). Kurve a zeigt einen direkten ELISA mit 1:120.000 verdünntem Kaninchenserum und 1:30.000 verdünntem TATP-Peroxidase-Tracer (TATP-Derivat-POD-Konjugat). Der Wendepunkt ist bei 1,6 µg L⁻¹; die mittlere Antikörperaffinität beträgt ca. 5,7 nM und die Nachweisgrenze liegt bei 92 ng L⁻¹ (n = 3). Kurve b zeigt einen direkten ELISA mit 1 : 120.000 verdünntem Kaninchenserum und 1 : 30.000 verdünntem TATP-Peroxidase-Tracer (TATP-Derivat-POD-Konjugat). Der Wendepunkt ist bei 0,98 µg L-¹; die mittlere Antikörperaffinität beträgt ca. 3,5 nM und die Nachweisgrenze liegt bei 170 ng L⁻¹ (n = 3).

### 1.5 Bestimmung der Kreuzreaktionen (Selektivität)

Mit den Seren der beiden Kaninchen aus Beispiel 1.4 wurden für die einzelnen Edukte der TATP-Derivat-Synthese (7-Oxooktansäure, H₂O₂ und Aceton) die Kreuzreaktivitäten relativ zu TATP bestimmt. Die Kreuzreaktivitäten wurden über den Quotienten der Wendepunkte (in µg L⁻¹) der sigmoidalen Kalibrierkurven ermittelt. Es zeigt sich in nachfolgender Tabelle, dass die polyklonalen Antikörper sehr selektiv sind. Die Ergebnisse wurden im direkten als auch indirekten ELISA erzielt.

**Tabelle:**

| | Kaninchen 1 | Kaninchen 2 |
|---|---|---|
| TATP | 100 % | 100 % |
| 7-Oxooktansäure | < 0,1 % | < 0,1 % |
| H₂O₂ | < 0,1 % | < 0,1 % |
| Aceton | < 0,1 % | < 0,1 % |

### Beispiel 2 (Vergleichsbeispiel):

### 2. DADP-Derivat-Synthese

Die Synthese erfolgte analog zur TATP-Synthese wie in Dubnikova et al. (Decomposition of triacteone triperoxide is an entropic explosion. Journal of the American Chemical Society 2005, 127(4), 1148).

## Patentansprüche

1. Triacetontriperoxid-Derivat gemäß der allgemeinen Formel (I), 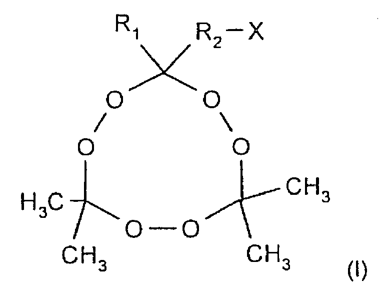 worin R₁ ein Wasserstoffrest oder eine optional halogenierte oder perhalogenierte C1-bis C5-Alkylgruppe ist, R₂ ein Linker-Molekül bedeutet und X eine reaktive oder reaktivierbare, kovalent oder nicht-kovalent an Proteine koppelbare Gruppe.

2. TATP-Derivat nach Anspruch 1, wobei R₂ ein gesättigter oder einfach oder mehrfach ungesättigter C1- bis C20-Alkylrest ist, ein Oligoethylenoxid, ein Peptid, ein Peptidoid, eine Nukleinsäure, ein Oligozucker, ein synthetisches oder natürliches Polymer oder Copolymer oder eine Kombinationen der vorgenannten Gruppen ist.

3. TATP-Derivat nach Anspruch 2, wobei R₂ ein unverzweigter gesättigter C1- bis C8-Alkylrest, insbesondere ein C2- bis C7-Alkylrest, vorzugsweise ein C5-Alkylrest, ist.

4. TATP-Derivat nach einem der vorhergehenden Ansprüche, wobei X eine Aminogruppe, eine Carboxylgruppe, eine Aldehydgruppe, eine Hydroxylgruppe, eine Thiolgruppe, eine Oxirangruppe, eine Aziridingruppe, eine Hydrazingruppe, eine Hydroxylamingruppe, eine Hydrazidgruppe, ein N-Hydroxysuccinimidester oder ein Salz, Ester oder Anhydrid von diesen bedeutet oder einer nicht-kovalent an Proteine koppelbaren Gruppe, insbesondere Biotin oder einem Derivat von diesem oder einem Peptid-Tag, insbesondere Oligo-Histidin, entspricht.

5. TATP-Derivat nach einem der vorhergehenden Ansprüche, wobei X einen Farbstoff, eine radioaktive Markierung oder eine stabile Isotopen-Markierung umfasst.

6. TATP-Derivat nach einem der vorhergehenden Ansprüche, wobei R₁ eine Methylgruppe ist.

7. TATP-Derivat nach einem der vorhergehenden Ansprüche, wobei das Triacetontriperoxid-Derivat 6-(3,6,6,9,9-Pentamethyl-1,2,4,5,7,8-hexaoxonan-3-yl)hexansäure gemäß der Formel (Ia) ist oder ein aktivierter Ester, ein Anhydrid oder Säurehalogenid von diesem

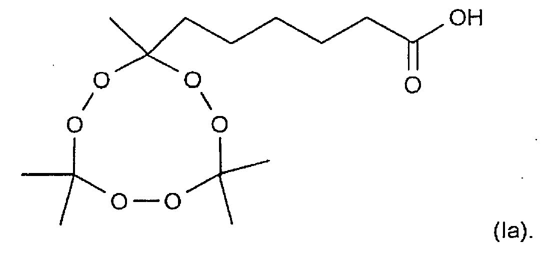

8. Verfahren zur Herstellung eines Triacetontriperoxid-Derivats nach einem der Ansprüche 1 bis 7 durch Umsetzung von Aceton, einem Aceton-Derivat oder einer Acetonabspaltenden Vorstufe mit Wasserstoffperoxid, einem Wasserstoffperoxid-Derivat oder einer Wasserstoffperoxid-abspaltenden Vorstufe mit einer Verbindung gemäß der allgemeinen Formel (III), 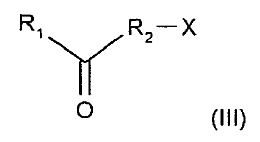 worin R₁, R₂ und X die oben genannten Bedeutungen haben.

9. Verfahren nach einem der Anspruch 8, wobei die Verbindung gemäß allgemeiner Formel (III) 7-Oxooktansäure gemäß Formel (IIIa) ist

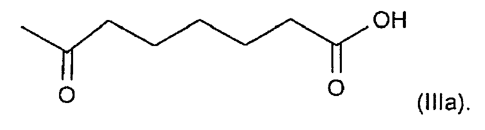

10. Verfahren nach einem der Ansprüche 8 oder 9, wobei die Reaktion in Gegenwart einer Säure durchgeführt wird oder autokatalytisch unter Verwendung eines saure Eigenschaften aufweisenden Edukts.

11. Verfahren nach einem der Ansprüche 8 bis 10, wobei Aceton, Wasserstoffperoxid und die Verbindung gemäß der allgemeinen Formel (III) in einem molaren Verhältnis von etwa 1 : 1 :1 eingesetzt werden.

12. Verwendung eines TATP-Derivats nach einem der Ansprüche 1 bis 7 zur kovalenten oder nicht-kovalenten Kopplung an Proteine, DNA oder Labels.

13. Verwendung eines TATP-Derivats nach einem der Ansprüche 1 bis 7 als Antigen zur Herstellung von TATP-spezifischen Bindern, insbesondere von monoklonalen oder polyklonalen Antikörpern, deren Fragmenten, Derivaten oder Fusionsprodukten, Antikörper-Analogen, Aptameren, Bindern auf Basis von Proteingerüsten, molekular geprägten Systemen, nanostrukturierten Materialien, selektiv bindenden Oberflächen oder isotopenmarkierten Verbindungen.

14. Verwendung nach Anspruch 13, wobei die TATP-spezifischen Binder in einem Affinitätssensor, in einem Affinitätsassay oder in einem affnitätsanreichernden System zum Nachweis von Triacetontriperoxid (TATP) eingesetzt werden.

15. Antikörper oder Antikörperfragment gegen ein Triacetontriperoxid-Derivat nach einem der Ansprüche 1 bis 7.

16. Sensor zum Nachweis von Triacetontriperoxid enthaltend einen TATP-spezifischen Binder nach Anspruch 13.

## Claims

1. A triacetone triperoxide derivative of general formula (I) 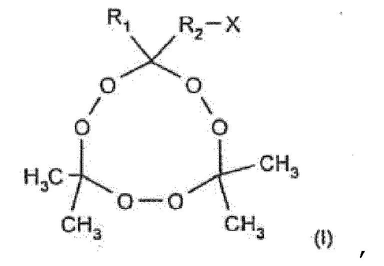 wherein R₁ is a hydrogen residue or an optionally halogenated or perhalogenated C1 to C5 alkyl group, R₂ represents a linker molecule, and X represents a reactive or reactivatable group that can be covalently or non-covalently bonded to proteins.

2. The TATP derivative according to Claim 1, wherein R₂ is a saturated or mono- or polyunsaturated C1 to C20 alkyl residue, an oligo(ethylene oxide), a peptide, a peptidoid, a nucleic acid, an oligosugar, a synthetic or natural polymer or copolymer, or a combination of the above-mentioned groups.

3. The TATP derivative according to Claim 2, wherein R₂ is an unbranched saturated C1 to C8 alkyl residue, in particular a C2 to C7 alkyl residue, preferably a C5 alkyl residue.

4. The TATP derivative according to one of the preceding claims, wherein X represents an amino group, a carboxyl group, an aldehyde group, a hydroxyl group, a thiol group, an oxirane group, an aziridine group, a hydrazine group, a hydroxylamine group, a hydrazide group, an N-hydroxysuccinimide ester or a salt, ester or anhydride thereof, or corresponds to a group that can be non-covalently coupled to proteins, in particular biotin or a derivative thereof, or a peptide tag, in particular oligohistidine.

5. The TATP derivative according to one of the preceding claims, wherein X comprises a dye, a radioactive label or a stable isotope label.

6. The TATP derivative according to one of the preceding claims, wherein R₁ is a methyl group.

7. The TATP derivative according to one of the preceding claims, wherein the triacetone triperoxide derivative is 6-(3,6,6,9,9-pentamethyl-1,2,4,5,7,8-hexaoxonan-3-yl)hexanoic acid according to formula (Ia) or an activated ester, an anhydride or acid halide thereof

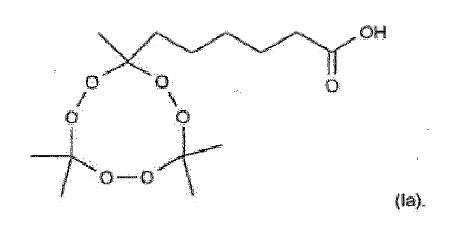

8. A method for preparing a triacetone triperoxide derivative according to one of Claims 1 to 7 by reacting acetone, an acetone derivative or an acetone-separating precursor with hydrogen peroxide, a hydrogen peroxide derivative or a hydrogen-peroxide-separating precursor and a compound according to general formula (III), 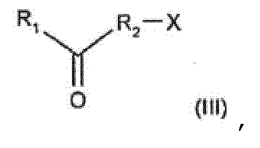 wherein R₁, R₂ and X have the meanings specified above.

9. The method according to Claim 8, wherein the compound according to general formula (III) is 7-oxooctanoic acid according to formula (IIIa)

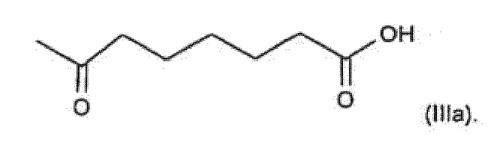

10. The method according to one of Claims 8 or 9, wherein the reaction is carried out in the presence of an acid, or autocatalytically with use of a reactant having acidic properties.

11. The method according to one of Claims 8 to 10, wherein acetone, hydrogen peroxide and the compound according to general formula (III) are used in a molar ratio of approximately 1:1:1.

12. Use of a TATP derivative according to one of Claims 1 to 7 for covalent or non-covalent coupling to proteins, DNA or labels.

13. Use of a TATP derivative according to one of Claims 1 to 7 as an antigen for producing TATP-specific binders, in particular monoclonal or polyclonal antibodies, fragments, derivatives or fusion products thereof, antibody analogues, aptamers, binders based on protein structures, molecularly imprinted systems, nanostructured materials, selectively binding surfaces or isotope-labelled compounds.

14. The use according to Claim 13, wherein the TATP-specific binders are used in an affinity sensor, an affinity assay or an affinity-accumulating system for detection of triacetone triperoxide (TATP).

15. An antibody or antibody fragment against a triacetone triperoxide derivative according to one of Claims 1 to 7.

16. A sensor for detecting triacetone triperoxide containing a TATP-specific binder according to Claim 13.

## Revendications

1. Dérivé de triperoxyde de triacétone selon la formule générale (I), 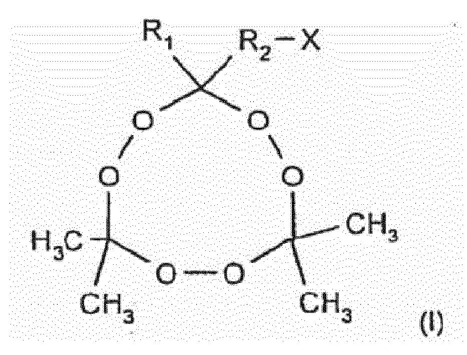 dans laquelle R₁ est un radical hydrogène ou un groupe alkyle en C₁ à C₅ optionnellement halogéné ou perhalogéné, R₂ est un groupe de liaison et X un groupe réactif ou réactivable pouvant être couplé de façon covalente ou non-covalente à des protéines

2. Dérivé de TATP selon la revendication 1, R₂ étant un radical alkyle en C₁ à C₂₀ saturé ou mono- ou polyinsaturé, un oligo-oxyde d'éthylène, un peptide, un peptidoïde, un acide nucléique, un oligosaccharide, un polymère ou copolymère synthétique ou naturel, ou une combinaison des groupes précités.

3. Dérivé de TATP selon la revendication 2, R₂ étant un radical alkyle en C₁ à C₈ saturé non-ramifié, notamment un radical alkyle en C₂ à C₇, de préférence un radical alkyle en C₅.

4. Dérivé de TATP selon l'une des revendications précédentes, X représentant un groupe amino, un groupe carboxyle, un groupe aldéhyde, un groupe hydroxyle, un groupe thiol, un groupe oxirane, un groupe azidirine, un groupe hydrazine, un groupe hydroxylamine, un groupe hydrazide, un ester de N-hydroxysuccinimide ou un de leurs sels, esters ou anhydrides ou correspondant à un groupe pouvant être couplé de façon non-covalente à des protéines, s'agissant notamment de la biotine ou d'un de ses dérivés ou d'un marqueur peptidique, notamment d'oligo-histidine.

5. Dérivé de TATP selon l'une des revendications précédentes, X comprenant un colorant, un marquage radioactif ou un marquage aux isotopes stables.

6. Dérivé de TATP selon l'une des revendications précédentes, R₁ étant un groupe méthyle.

7. Dérivé de TATP selon l'une des revendications précédentes, le dérivé de triperoxyde de triacétone étant de l'acide 6-(3,6,6,9,9-pentamethyl-1,2,4,5,7,8-hexaoxonane-3-yl)hexanoïque selon la formule (Ia) ou l'un des ses esters activés, ses anhydrides ou ses halogénures d'acyle

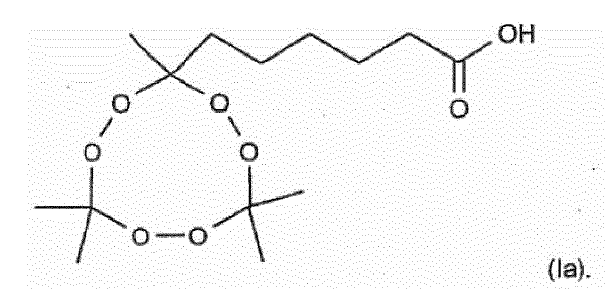

8. Procédé de préparation d'un dérivé de triperoxyde de triacétone selon l'une des revendications 1 à 7, en faisant réagir l'acétone, un dérivé d'acétone ou un précurseur pouvant libérer de l'acétone avec le peroxyde d'hydrogène, un dérivé de peroxyde d'hydrogène ou un précurseur pouvant libérer du peroxyde d'hydrogène avec un composé selon la formule générale (III), 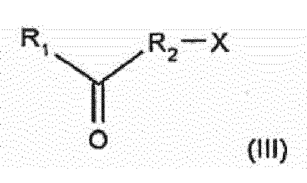 dans laquelle R₁, R₂ et X ont les significations indiquées ci-dessus.

9. Procédé selon la revendication 8, le composé selon la formule générale (III) étant de l'acide 7-oxoctanoïque selon la formule (IIIa)

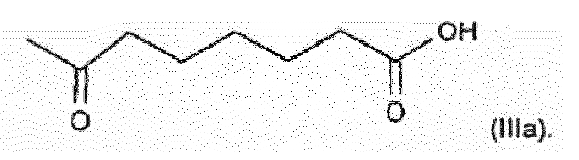

10. Procédé selon l'une des revendications 8 ou 9, ladite réaction étant réalisée en présence d'un acide ou de manière autocatalytique en mettant en oeuvre un produit de départ ayant des propriétés acides.

11. Procédé selon l'une des revendications 8 à 10, l'acétone, le peroxyde d'hydrogène et le composé selon la formule générale (III) étant mis en oeuvre dans un rapport molaire d'environ 1 : 1 : 1.

12. Utilisation d'un dérivé de TATP selon l'une des revendications 1 à 7 pour réaliser un couplage covalent ou non-covalent à des protéines, à l'ADN ou à des marqueurs.

13. Utilisation d'un dérivé de TATP selon l'une des revendications 1 à 7 en tant qu'antigène afin de préparer des éléments de liaison spécifiques au TATP, s'agissant notamment d'anticorps monoclonaux ou polyclonaux, de leur fragments, dérivés ou produits de fusion, d'éléments analogues à des anticorps, d'aptamères, d'éléments de liaison à base de structures protéiniques, de systèmes pourvus d'un marquage moléculaire, de matériaux nanostructurés, de surfaces permettant d'établir des liaisons spécifiques ou de composés marqués aux isotopes.

14. Utilisation selon la revendication 13, les éléments de liaison spécifiques au TATP étant mis en oeuvre dans un capteur d'affinité, dans un système de dosage par affinité ou dans un système à enrichissement d'affinité destiné à la détection de triperoxyde de triacétone (TATP).

15. Anticorps ou fragment d'anticorps ciblant un dérivé de triperoxyde de triacétone selon l'une des revendications 1 à 7.

16. Capteur destiné à la détection de triperoxyde de triacétone, contenant un élément de liaison spécifique au TATP selon la revendication 13.
